# EUROPEAN PATENT SPECIFICATION

(11) **EP 1 100 409 B1**
(45) Date of publication and mention of the grant of the patent: **16.03.2005**
(21) Application number: 99934433.6
(22) Date of filing: 29.07.1999
(51) Int. Cl.: A61F 2/06

(54) **SMALL VESSEL EXPANDABLE STENT**
EXPANDIERBARER STENT FÜR KLEINLUMIGE GEFÄSSE
STENT EXTENSIBLE POUR PETITS VAISSEAUX

(30) Priority: 31.07.1998 US 94809 P
(43) Date of publication of application: 23.05.2001
(73) Proprietor: EVYSIO MEDICAL DEVICES ULC, Vancouver, British Columbia V5Z 1L7 (CA)
(72) Inventor: RICCI, Donald, R., Vancouver, British Columbia V6R 1M9 (CA); SHUKOV, George, A., Los Altos Hills, CA 94022 (US); PENN, Ian, M., Vancouver, British Columbia V6R 4E9 (CA); DICKSON, Todd, Mountain View, CA 94043 (US)
(74) Representative: Plougmann & Vingtoft A/S
(86) International application number: PCT/CA1999/000694
(87) International publication number: WO 2000/007522

(56) References cited:
- EP-A- 0 689 805
- DE-A- 19 722 384
- US-A- 5 749 851
- US-A- 5 776 161

## Description

### TECHNICAL FIELD

The present invention relates to an expandable stent and to a method for production of same. More particularly, the present invention relates to an expandable stent particularly suited for deployment in a small diameter body passageway (e.g., a lumen or artery having a diameter of less than or equal to about 3 mm).

### BACKGROUND ART

Stents are generally known. Indeed, the term "stent" has been used interchangeably with terms such as "intraluminal vascular graft" and "expansible prosthesis". As used throughout this specification the term "stent" is intended to have a broad meaning and encompasses any expandable prosthetic device for implantation in a body passageway (e.g., a lumen or artery).

In the past ten years, the use of stents has attracted an increasing amount of attention due the potential of these devices to be used, in certain cases, as an alternative to surgery. Generally, a stent is used to obtain and maintain the patency of the body passageway while maintaining the integrity of the passageway. As used in this specification, the term "body passageway" is intended to have a broad meaning and encompasses any duct (e.g., natural or iatrogenic) within the human body and can include a member selected from the group comprising: blood vessels, respiratory ducts, gastrointestinal ducts and the like.

Stent development has evolved to the point where the vast majority of currently available stents rely on controlled plastic deformation of the entire structure of the stent at the target body passageway so that only sufficient force to maintain the patency of the body passageway is applied during expansion of the stent.

Generally, in many of these systems, a stent, in association with a balloon, is delivered to the target area of the body passageway by a catheter system. Once the stent has been properly located (for example, for intravascular implantation the target area of the vessel can be filled with a contrast medium to facilitate visualization during fluoroscopy), the balloon is expanded thereby plastically deforming the entire structure of the stent so that the latter is urged in place against the body passageway. As indicated above, the amount of force applied is at least that necessary to expand the stent (i.e., the applied the force exceeds the minimum force above which the stent material will undergo plastic deformation) while maintaining the patency of the body passageway. At this point, the balloon is deflated and withdrawn within the catheter, and is subsequently removed. Ideally, the stent will remain in place and maintain the target area of the body passageway substantially free of blockage (or narrowing).

See, for example, any of the following patents:
United States patent 4,733,665 (Palmaz),
United States patent 4,739,762 (Palmaz),
United States patent 4,800,882 (Gianturco),
United States patent 4,907,336 (Gianturco),
United States patent 5,035,706 (Gianturco et al.),
United States patent 5,037,392 (Hillstead),
United States patent 5,041,126 (Gianturco),
United States patent 5,102,417 (Palmaz),
United States patent 5,147,385 (Beck et al.),
United States patent 5,282,824 (Gianturco),
United States patent 5,316,023 (Palmaz et al.),
Canadian patent 1,239,755 (Wallsten),
Canadian patent 1,245,527 (Gianturco et al.),
Canadian patent application number 2,134,997 (Penn et al.),
Canadian patent application number 2,171,047 (Penn et al.),
Canadian patent application number 2,175,722 (Penn et al.),
Canadian patent application number 2,185,740 (Penn et al.),
Canadian patent application number 2,192,520 (Penn et al.),
International patent application PCT/CA97/00151 (Penn et al.),
International patent application PCT/CA97/00152 (Penn et al.), and
International patent application PCT/CA97/00294 (Penn et al.),
for a discussion on previous stent designs and deployment systems.

Published International patent application WO 95/26695 [Lau et al. (Lau)] teaches a self-expandable, foldable stent which may be delivered using a catheter or other technique. The purported point of novelty in Lau relates to a stent which may be folded along its longitudinal axis. The folding is accomplished by conferring bending and twisting stresses to the stent, which stresses, for the material used to produce the stent, do not exceed that minimum stresses above which plastic deformation of the stent will occur - i.e., application of these stresses to the stent results in the storage of mechanical energy in the stent but does not result in the occurrence of any plastic deformation.

United States patent 5,643,312 [Fischell et al. (Fischell)] teaches a stent having a multiplicity of closed circular structures connected by a series of longitudinals. The stent is initially produced in a pre-deployment form comprising ovals connected by the longitudinals (see Figures 4 and 5). The pre-deployment form of the stent is than placed on the end of a balloon stent delivery catheter and the ovals are folded about their minor axis by securing the ovals at each end of the structure and translating a pair of opposed longitudinals (see Figure 6).

Many conventional stents rely on plastic deformation of the material from the stent is constructed for proper deployment. The stress-strain profile is substantially consistent for a given material (e.g., stainless steel) from which the stent is constructed. This profile has two specific regions of interest.

The first region is that portion of the profile in which the material exhibits elastic behaviour. Specifically, the profile is substantially linear (i.e, a substantially constant slope). During this first region, if the expansive force is removed, the stent will recoil to near its original diameter - i.e., the material is still in an elastic state which results in recoil of the stent if the force is removed.

The second region of the profile is reached when the occurrence of recoil is substantially reduced - i.e., the stent will recoil less than 15%, preferably less than 10%, more preferably less than 5%, of the expanded diameter of the stent. Practically, this is the elastic limit of the material. Once this point is reached, the material begins to exhibit plastic behaviour. This second region of the profile has three successive sub-regions of interest along the profile: (i) plastic deformation or yielding; (ii) strain hardening; and (iii) necking.

Once the material begins to exhibit plastic behaviour, continuation of the expansive forces results in a breakdown of the material which causes it to deform permanently - this is known as "plastic deformation" or "yielding". In this sub-region, the stent will continue to expand with substantially no increase in the expansive forces. The term "maximum yield point", when used throughout this specification in the context of an expanding stent, is intended to mean the point on the profile above which an increased expansive force can be applied to the stent to further expand the stent resulting in a decrease in the cross-sectional area of the expanding material. In other words, above the "maximum yield point", the onset of "strain hardening" sub-region of the profile occurs in which the profile rises as a continuous curve to a maximum stress which is also known as the "ultimate stress". Exceeding the ultimate stress leads to onset of "necking" sub-region of the profile in which the cross-sectional area of the stent material decreases in a localized region of the stent. Since the cross-section area is decreasing, the smaller area can only carry a decreasing load resulting in a downward curve from the ultimate stress until the material breaks at the "fracture stress".

Thus, in conventional stents which rely on plastic deformation for deployment, the unexpanded stent is typically in an elastic state (i.e., the first region of the above-mentioned profile) and is expanded to a plastic state (i.e., the second region of the above-mentioned profile), particularly to a point falling with the first sub-region of the latter. Practically, it is generally desirable to deploy the stent to a diameter which is as close as possible to, but does not exceed, the maximum yield point discussed above. The reason for is that the radial rigidity of the stent is maximized.

One application of stenting which has received little or no attention is that of stenting of small diameter body passageways. As used throughout this specification, the term "small diameter body passageway" is intended to mean an artery or lumen having a diameter of less than or equal to about 3 mm. As used throughout this specification, the term "large diameter body passageway" is intended to mean an artery or lumen having a diameter of greater than about 3 mm.

One likely reason for this is that the conventional stents are designed for implantation into a large diameter body passageway. More specifically, stents which rely on controlled plastic deformation of the entire structure of the stent at the target body passageway are designed to have a maximum yield point at an expansion diameter commensurate with the large diameter body passageway. For most conventional such stents the maximum yield point is reached at a point when the diameter of the expanded stent is about 4 mm to about 5 mm. In the unexpanded state, the diameter of the stent is about 1.5 mm. Such stents are inappropriate for implantation into a small body passageway - i.e., an artery or lumen having a diameter of less than or equal to about 3 mm. This principal reason for this is the dual effect of relatively high recoil forces inherent with such body passageways and poor radial rigidity of the stent if it is expanded to a diameter of less than or equal to about 3 mm - i.e., well below the maximum yield point.

Accordingly, it would be desirable to have an improved stent which overcomes these disadvantages. It would be further desirable if the improved stent could be manufactured readily. It would be further desirable if the improved stent could be deployed using conventional stent delivery systems.

### DISCLOSURE OF THE INVENTION

It is an object of the present invention to provide a novel expandable stent which obviates or mitigates at least one of the above-mentioned disadvantages of the prior art.

Thus, in one of its aspects, the present invention provides an unexpanded stent comprising a proximal end and a distal end in communication with one another, a tubular wall disposed between the proximal end and the distal end, the tubular wall having a longitudinal axis and a porous surface defined by a plurality of interconnecting struts, the stent being expandable upon the application of a radially outward force thereon to undergo plastic deformation to a maximum yield point when the tubular wall has a diameter of less than or equal to about 3.5 mm.

In another of its aspects, the present invention provides an unexpanded stent comprising a proximal end and a distal end in communication with one another, a tubular wall disposed between the proximal end and the distal end, the tubular wall having a longitudinal axis and a porous surface defined by a plurality of interconnecting struts, the stent being expandable upon the application of a radially outward force thereon:
from a first unexpanded position to a second pre-expanded position at which the stent has reached a point of plastic deformation; and
from the second pre-expanded position to a third expanded position wherein the stent will undergo plastic deformation to maximum yield point when the tubular wall has a diameter of less than or equal to about 3.5 mm.

In another of its aspects, the present invention- provides a partially expanded stent comprising a proximal end and a distal end in communication with one another, a tubular wall disposed between the proximal end and the distal end, the tubular wall having a longitudinal axis and a porous surface defined by a plurality of interconnecting struts, the stent:
having been expanded by the application of a radially outward force thereon from a first unexpanded position to a second pre-expanded position at which the stent has reached a point of plastic deformation, and
being further expandable upon the application of a radially outward force thereon from the second pre-expanded position to a third expanded position wherein the stent will undergo plastic deformation to a maximum yield point. Preferably (but not necessarily), in the third expanded position of the stent, the maximum yield point is reached when the tubular wall has a diameter of less than or equal to about 3.5 mm.

In yet another of its aspects, the present invention provides a stent delivery kit comprising:
a catheter;
an expandable member disposed on the catheter; and
a partially expanded stent disposed on the catheter, the stent comprising a proximal end and a distal end in communication with one another, a tubular wall disposed between the proximal end and the distal end, the tubular wall having a longitudinal axis and a porous surface defined by a plurality of interconnecting struts, the stent:
   having been expanded by the application of a radially outward force thereon from a first unexpanded position to a second pre-expanded position at which the stent has reached a point of plastic deformation, and
   being expandable upon the application of a radially outward force thereon from the second pre-expanded position to a third expanded position wherein the stent will undergo plastic deformation to a maximum yield point. Preferably (but not necessarily), in the third expanded position of the stent, the maximum yield point is reached when the tubular wall has a diameter of less than or equal to about 3.5 mm.

In yet another if its aspects, the present invention provides a method for mounting an unexpanded stent on a catheter having an expandable member disposed thereon, the unexpanded stent comprising a proximal end and a distal end in communication with one another, a tubular wall disposed between the proximal end and the distal end, the tubular wall having a longitudinal axis and a porous surface defined by a plurality of interconnecting struts, the stent being expandable upon the application of a radially outward force thereon:
(i) expanding the unexpanded stent to a second pre-expanded position at which the stent has reached a point ofplastic deformation to produce a partially expanded stent; and
(ii) placing the partially expanded stent on the expandable member of the catheter.

Thus, the present inventors have developed a novel stent which is fundamentally different from stents produced heretofore. Specifically, whereas conventional stents are normally deployed by a single expansion from an initial unexpanded state in which the stent material exhibits elastic behaviour to a fully (i.e., final) expanded state in which the stent exhibits plastic behaviour, the approach in a preferred embodiment of the present invention is based on delivery of a stent which has been pre-expanded to a point of plastic deformation prior to deployment in a subject. Thus, a preferred embodiment of the present stent is deployed using two distinct expansion steps. As will be developed below, in this preferred embodiment, the initial expansion step is conducted *ex vivo* whereas the final expansion step is conducted *in vivo.*

While there are many advantages which accrue from the present stent, the three principal advantages are:
(i) debulking of the stent - i.e., since the stent is deployed *in vivo* from a pre-expanded state, less material is required to construct the unexpanded stent when compared to an unexpanded stent which is deployed *in vivo* - i.e., the stent may be constructed from a small tube;
(ii) since the amount of material need to construct the stent is reduced, the flexibility of the stent is improved; and/or
(iii) the ability to provide a stent having a maximum yield point which is reached at a diameter commensurate (i.e., relative to the prior art) with the typical diameter of a small body passageway.

While the present stent is suited to use with both large body passageways and small body passageways, it is particularly well suited for the latter. From a practical perspective, much of the state of the art in stenting utilizes a low profile balloon mounted on a catheter. Conventionally, this balloon can have a diameter of about 1.0 mm to about 1.3 mm. Since most conventional stents have a final expanded diameter to unexpanded diameter ratio of about 3.0 (this assures that the point of plastic deformation has been reached and the stent material has been stressed to near the maximum yield point), a conventional stent has a final expansion diameter of 4.5-5.0 mm, making it unsuitable for use in a small diameter body passageway.

A novel application of the present stent is to produce the unexpanded stent with a small enough diameter such that the optimal expanded diameter to unexpanded diameter ratio can be reconciled with the desire that the stent material reaches the maximum yield point once the stent is expanded to a diameter of less than or equal to about 3.5 mm, preferably less than or equal to about 3.3 mm, more preferably in the range of from about 2.2 mm to about 3.3 mm, most preferably in the range of from about 2.5 mm to about 3.0 mm. Practically, this has been achieved by designing the stent so that it may be initially expanded to allow it to be mounted on a delivery system - e.g., a conventional low profile balloon mounted on a catheter. Once so mounted, the stent is delivery to the target small diameter body passageway whereupon a second and distinct expansion step is effected to deploy the stent. Thus, as stated above, in a preferred embodiment of the present stent, the initial expansion step is conducted *ex vivo* and the final expansion step is conduct *in vivo.*

To the knowledge of the present inventors, this is the first targeted approach to stenting of small diameter body passageways.

### BRIEF DESCRIPTION OF THE DRAWINGS

Embodiments of the present invention will be described with reference to the accompanying drawings wherein like numerals designate like parts and in which:

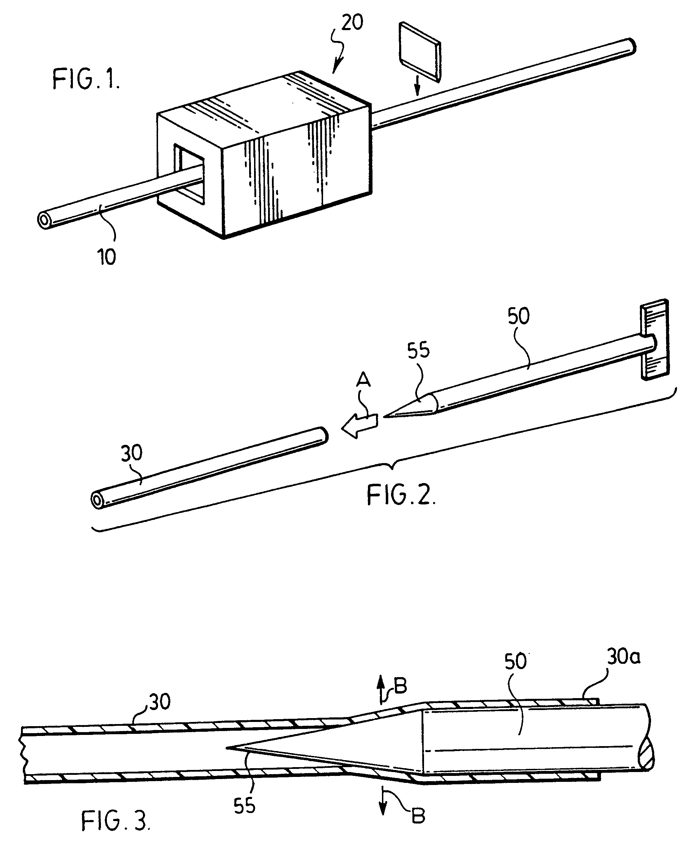

Figure 1 illustrates a schematic of an embodiment for producing the present stent in its unexpanded state;
Figure 2 and 3 illustrate a schematic of one preferred embodiment of effecting partial expansion of the unexpanded stent;

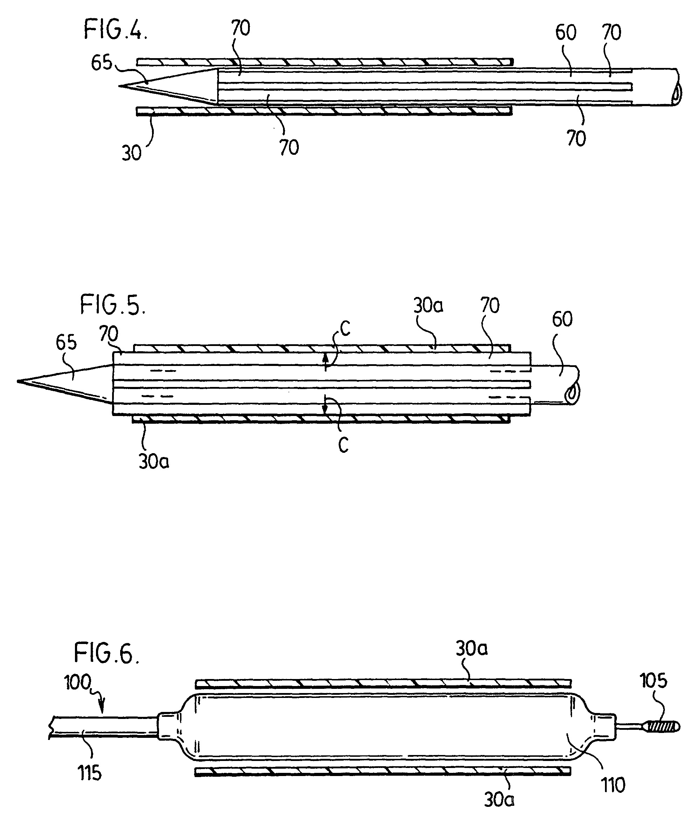

Figures 4 and 5 illustrate a schematic of another preferred embodiment of effecting partial expansion of the unexpanded stent;

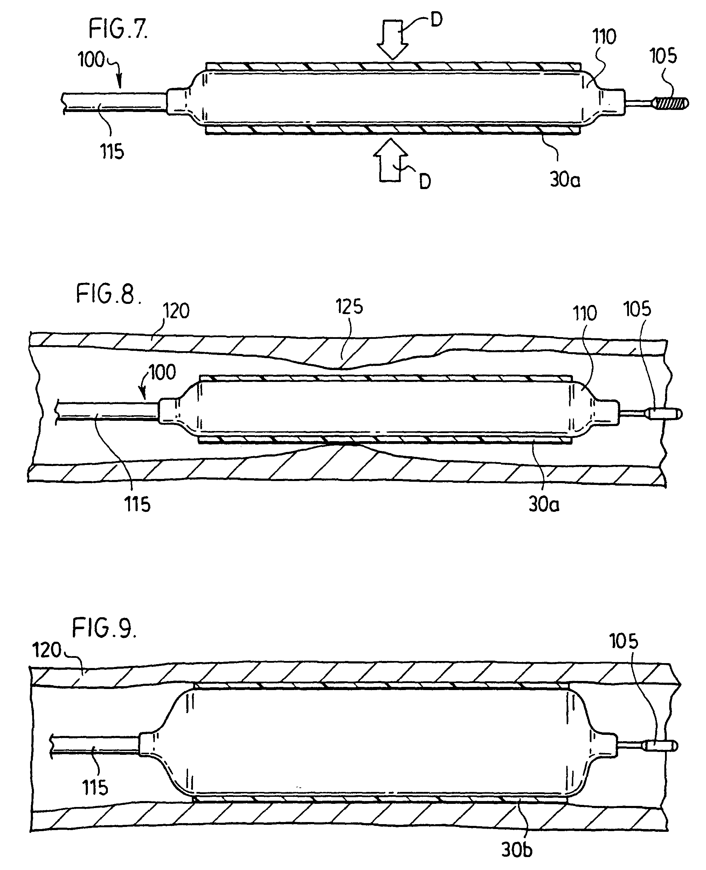

Figures 6 and 7 illustrate a schematic of mounting of the partially expanded stent produced in Figures 2-5 on to a balloon-mounted catheter;
Figure 8 illustrates the placement in a small diameter body passageway the balloon-mounted catheter have mounted thereon the partially expanded stent; and
Figure 9 illustrates final expansion of the partially expanded stent in the small diameter body passageway.

### BEST MODE FOR CARRYING OUT THE INVENTION

The specific design of the porous surface is not particularly restricted. Preferably, in the unexpanded state, at least two of the struts meet at the intersection point to define an acute angle.

In the context of the present stent, various repeating patterns in the porous surface of the tubular wall are particularly advantageous. Generally, the preferred repeating pattern is a plurality of intersecting members arranged to define a first repeating pattern comprised of a polygon having a pair of side walls substantially parallel to the longitudinal axis, a concave-shaped first wall having a first apex and a convex-shaped second wall having a second apex, the first wall and the second wall connecting the side walls. Preferably, at least one, more preferably both, of the first apex and the second apex is substantially flat. The first apex and the second apex may be of the same or different length.

Preferably, the side walls comprise longitudinal struts disposed substantially parallel to the longitudinal axis of the stent, each of the longitudinal struts comprising flexure means for substantially complementary extension and compression of a diametrically opposed pair of the longitudinal struts upon flexure of the stent. Practically, the flexure means may comprise at least one lateral section disposed in each longitudinal strut. The lateral section may have a pointed apex, a rounded apex, a flat apex and the like. Further, the flexure means may comprise more than one such lateral per longitudinal strut. If two sections are provided per longitudinal strut, they may be symmetric or asymmetric. Further the two sections may have substantially the same shape and differing size, or they may have differing shape and size, or they may have substantially the same shape and differing size.

The preferred flexure means comprises an S-shaped portion - e.g., a pair of joined curved sections wherein each curve section has an arc equal to or greater that about 180°. The curved sections may be of substantially the same or different size.

Non-limiting examples of various preferred repeating patterns incorporating some or all of these features and which are useful in the context of the present stent are disclosed in the following copending patent applications:
Canadian patent application number 2,134,997 (Penn et al.),
Canadian patent application number 2,171,047 (Penn et al.),
Canadian patent application number 2,175,722 (Penn et al.),
Canadian patent application number 2,185,740 (Penn et al.),
Canadian patent application number 2,192,520 (Penn et al.),
International patent application PCT/CA97/00151 (Penn et al.) and
International patent application PCT/CA97/00152 (Penn et al.).
Of course, many conventional repeating patterns may be incorporated into the present stent.

The present stent may be constructed from any suitable starting material. Preferably, the starting material is a thin tube of a metal or alloy. In one preferred embodiment, the starting material may be one which is plastically deformable-non-limiting examples of such a material include stainless steel, titanium, tantalum and the like. In another preferred embodiment, the starting material may be one which expands via temperature-dependent memory (i.e., a material which will expand upon reaching a certain temperature) - non-limiting examples of such a material include balloon expandable nitinol and the like.

With reference to Figure 1, there is illustrated a side elevation of a solid tube 10 of a starting material for producing the present stent. The nature of solid tube 10 is not particularly restricted and includes all materials conventionally used to produce stents. In one preferred embodiment, solid tube 10 is constructed of a plastically deformable material. As discussed above, a non-limiting example of such a material is stainless steel. In another preferred embodiment, solid tube 10 is constructed of a material which will expand when a certain temperature is reached. In this embodiment, the material may be a metal alloy (e.g., nitinol) capable of self-expansion at a temperature of at least about 30°C, preferably in the range of from about 30° to about 40°C. Preferably, solid tube 10 has a thickness in the range of from about 0.076 mm to about 0.38 mm [from about 0.003 to about 0.015 inches].

If the stent is to be used in a small body passageway, it is preferred that solid tube 10 have a diameter in the range of from about 0.5 mm to about 1.0 mm. If the stent is to be used in a large body passageway, it is preferred that solid tube have a diameter of greater than about 1.0 mm, preferably in the range of from about 1.3 mm to about 1.6 mm.

Solid tube 10 is then subjected to processing which results in removal of a portion thereof to define a porous surface. While the precise nature of this processing is not particularly restricted, it is preferred that the processing by effected on a computer programmable, laser cutting system illustrated generally at 20. Laser cutting system 20 operates by:
(i) receiving solid tube 10;
(ii) moving solid tube 10 longitudinally and rotationally under a laser beam to selectively remove sections of solid tube 10 thereby defining a porous surface; and
(iii) cutting stent sections of desirable length of solid tube 10.
A suitable such laser cutting system is known in the art as the LPLS-100 Series Stent Cutting Machine. The operation of this system to produce the unexpanded stent is within the purview of a person skilled in the art.

Thus, the stent produced from laser cutting system 20 is in the unexpanded state - i.e., the stent will exhibit elastic behaviour in this state.

If desired, the stent may be subjected to further processing to apply a coating material thereon. The coating material may be disposed continuously or discontinuously on the surface of the stent. Further, the coating may be disposed on the interior and/or the exterior surface(s) of the stent. The coating material may be one or more of a biologically inert material (e.g., to reduce the thrombogenicity of the stent), a medicinal composition which leaches into the wall of the body passageway after implantation (e.g., to provide anticoagulant action, to deliver a pharmaceutical to the body passageway and the like), a radioactive composition (e.g., to render the stent radioopaque during delivery) and the like.

The stent is preferably provided with a biocompatible coating, in order to minimize adverse interaction with the walls of the body vessel and/or with the liquid, usually blood, flowing through the vessel. The coating is preferably a polymeric material, which is generally provided by applying to the stent a solution or dispersion of preformed polymer in a solvent and removing the solvent. Non-polymeric coating material may alternatively be used. Suitable coating materials, for instance polymers, may be polytetraflouroethylene or silicone rubbers, or polyurethanes which are known to be biocompatible. Preferably, however, the polymer has zwitterionic pendant groups, generally ammonium phosphate ester groups, for instance phosphoryl choline groups or analogues thereof. Examples of suitable polymers are described in published International patent applications WO-A-93/16479 and WO-A-93/15775. Polymers described in those specifications are hemo-compatible as well as generally biocompatible and, in addition, are lubricious. When a biocompatible coating is used, It is important to ensure that the surfaces of the stent are completely coated in order to minimize unfavourable interactions, for instance with blood, which might lead to thrombosis.

This good coating can be achieved by suitable selection of coating conditions, such as coating solution viscosity, coating technique and/or solvent removal step. The coating, if present, can be applied to the stent in the pre-expanded or contracted state. Preferably, the stent is applied to the coating in the pre-expanded state to obviate or mitigate the likelihood of damage to the coating during the transition from the contracted (i.e., unexpanded) state to the pre-expanded stated.

With reference to Figures 2 and 3, there is illustrated, in schematic, one preferred embodiment of effecting partial expansion of the unexpanded stent produced in Figure 1. Specifically, there is shown a solid mandrel 50 having a tapered tip 55, and an unexpanded stent 30 produced from laser cutting system 20 (Figure 1). Tapered tip 55 of mandrel 50 is placed one open end of unexpanded stent 30. Thereafter, mandrel 50 is forced into stent 30 in the direction of arrow A. Practically, this can be achieved by keeping mandrel 50 stationary and forcing stent 30 over mandrel 50 or by keep stent 30 stationary and forcing mandrel 50 into stent 30. With reference to Figure 3, as mandrel 50 enters stent 30, tapered tip 55 serves to apply a radially outward force in the direction of arrows B on stent 30 of sufficient magnitude such that the stent reaches the point of plastic deformation - i.e., when mandrel 50 is removed, the partially expanded stent will recoil less that 15%, preferably less than 10%, more preferably less than 5%, of the expanded diameter of the stent. The partially expanded stent is designated 30a.

With reference to Figures 4 and 5, there is illustrated, in schematic, of one preferred embodiment of effecting partial expansion of the unexpanded stent produced in Figure 1. Specifically, there is shown an expandable mandrel 60 having a tapered tip 65 and a series of expandable vanes 70. The diameter of expandable mandrel 60 (in the unexpanded state) is less than that of unexpanded stent 30. Thus, expandable mandrel 60 may be readily placed with stent 30 as illustrated in Figure 4. Once expandable mandrel 60 is placed within stent 30 (preferably the ends of expandable mandrel 60 emanate from the ends of stent 30), the interior of the expandable mandrel is pressurized (e.g., by forcing a fluid into the interior of expandable mandrel 60 or similar means) resulting in application of a radially outward force in the direction of arrows C on stent 30 of sufficient magnitude such that the stent reaches the point of plastic deformation-i.e., when mandrel 60 is removed, the partially expanded stent will recoil less that 15%, preferably less than 10%, more preferably less than 5%, of the expanded diameter of the stent. The partially expanded stent is designated 30a. After partial expansion of stent 30, expandable mandrel 60 is deflated and withdrawn from partially expanded stent 30a. If the stent is to be used in a small body passageway, it is preferred that the diameter of the partially expanded stent be at least about 1.3 mm, preferably in the range of from about 1.4 mm to about 1.6 mm - i.e., of sufficient diameter to be placed on an expandable member of catheter delivery system.

With reference to Figures 6 and 7, there is illustrated a schematic of mounting of the partially expanded stent produced in Figures 2-5 (or by any other means) on to a balloon-mounted catheter 100. Catheter 100 comprises a balloon 110 mount at a distal end thereof. Balloon 110 is in communication with a tube 115. A guidewire 105 is disposed coaxially within balloon 110 and tube 115. Catheter 100 is conventional and may be, for example, a low profile balloon as discussed above. Balloon 110 is placed within partially expanded stent 30a and crimped (e.g., mechanically) down on balloon 110 in the direction of arrows D (Figure 7). Such mounting of a stents on a balloon is conventional.

Deployment of partially expanded stent 30a will now be described with reference to Figures 8 and 9. Figure 8 illustrates the placement in a small diameter body passageway of the balloon-mounted catheter have mounted thereon the partially expanded stent. Figure 9 illustrates final expansion of the partially expanded stent in the small diameter body passageway.

Thus, there is illustrated a small body passageway 120 have a stenosis 125 therein. Catheter 100 having partially expanded stent 30a mounted thereon is navigated to the stenosis in a conventional manner. On in the proper location, balloon 110 is pressurized (e.g., by forcing a fluid through tube 115 into balloon 100) resulting in the application of a radially outward force on partially expanded stent 30a. Exertion of this force results in further plastic deformation of partially expanded stent 30a until it reaches its fully expanded state 30b at which point stenosis 125 is alleviated - at this fully expanded state, the stent material is close to, but has not exceed, the maximum yield point.

As will be apparent to those of skill in the art, it is important to ensure that, during expansion of partially expanded stent 30a to its fully expanded state 30b, the maximum yield point is not exceeded since this can results in catastrophic failure of the stent. In other words, expansion of the stent should not be at a stress-strain level in the beyond the first sub-region of the second region of the profile discussed above. With the present specification in hand, those of skill in the art will be readily able to reconcile the following factors to produce a useful stent: (i) specific porous design of the stent, (ii) the material used to construct the stent, (iii) the diameter of tubular material from which the unexpanded stent is made, (iv) stress-strain level at which the point of plastic deformation is reached and (v) stress-strain level at which the maximum yield point is reached.

While this invention has been described with reference to illustrative embodiments, this description is not intended to be construed in a limiting sense. Various modifications of the illustrative embodiments, as well as other embodiments of the invention, will be apparent to persons skilled in the art upon reference to this description. It is therefore contemplated that the appended claims will cover any such modifications or embodiments.

## Claims

1. An unexpanded stent comprising a proximal end and a distal end in communication with one another, a tubular wall disposed between the proximal end and the distal end, the tubular wall having a longitudinal axis and a porous surface defined by a plurality of interconnecting struts, **characterized in that** the stent is expandable upon the application of a radially outward force thereon to undergo plastic deformation to a maximum yield point when the tubular wall has a diameter of less than or equal to about 3.5 mm.

2. The unexpanded stent defined in Claim 1, wherein the stent is expandable upon the application of a radially outward force thereon to undergo plastic deformation to a maximum yield point when the tubular wall has a diameter of less than or equal to about 3.3 mm.

3. The unexpanded stent defined in Claim 1, wherein the stent is expandable upon the application of a radially outward force thereon to undergo plastic deformation to a maximum yield point when the tubular wall has a diameter in the range of from about 2.2 to about 3.3 mm.

4. The unexpanded stent defined in Claim 1, wherein the stent is expandable upon the application of a radially outward force thereon to undergo plastic deformation to a maximum yield point when the tubular wall has a diameter in the range of from about 2.5 to about 3.0 mm.

5. The unexpanded stent defined in any one of Claims 1-4, wherein the stent has a diameter less than or equal to about 1.1 mm.

6. The unexpanded stent defined in any one of Claims 1-4, wherein the stent has a diameter in the range of from about 0.5 to about to about 1.1 mm.

7. The unexpanded stent defined in any one of Claims 1-4, wherein the stent has a diameter in the range of from about 0.5 to about to about 1.0 mm.

8. The unexpanded stent defined in any one of Claims 1-7, wherein the tubular wall has a substantially circular cross-section.

9. The unexpanded stent defined in any one of Claims 1-8, wherein the tubular wall is constructed of a plastically deformable material.

10. The unexpanded stent defined in any one of Claims 1-9, wherein the stent has disposed thereon a coating material selected from one or more of a biologically inert material, a medicinal composition and a radioactive composition.

11. A partially expanded stent comprising a proximal end and a distal end in communication with one another, a tubular wall disposed between the proximal end and the distal end, the tubular wall having a longitudinal axis and a porous surface defined by a plurality of interconnecting struts, **characterized in that** the stent:
has been expanded by the application of a radially outward force thereon from a first unexpanded position to a second pre-expanded position at which the stent has reached a point of plastic deformation, and
is further expandable upon the application of a radially outward force thereon from the second pre-expanded position to a third expanded position wherein the stent will undergo plastic deformation to a maximum yield point when the tubular wall has a diameter of less than or equal to about 3.5 mm.

12. The partially expanded stent defined in Claim 11, wherein, in the third expanded position of the stent, the maximum yield point is reached when the tubular wall has a diameter of less than or equal to about 3.3 mm.

13. The partially expanded stent defined in Claim 11, wherein, in the third expanded position of the stent, the maximum yield point is reached when the tubular wall has a diameter in the range of from about 2.2 to about 3.3 mm.

14. The partially expanded stent defined in Claim 11, wherein, in the third expanded position of the stent, the maximum yield point is reached when the tubular wall has a diameter in the range of from about 2.5 to about 3.0 mm.

15. A stent delivery kit comprising:
a catheter;
an expandable member disposed on the catheter; and
the partially expanded stent defined in any one of Claims 11-14 disposed on the catheter.

16. The stent delivery kit defined in Claim 15, wherein the stent is mechanically mounted on the expandable member.

17. The stent delivery kit defined in Claim 16, wherein the stent is crimped onto the expandable member.

## Patentansprüche

1. Ein unausgedehnter Stent, der ein nahes und ein distales Ende umfasst, die miteinander in Verbindung stehen, eine hülsenförmige Wand, die sich zwischen dem nahen und dem distalen Ende befindet, und die eine Längsachse hat sowie eine poröse Oberfläche, gebildet durch eine Mehrzahl von Verbindungsstreben, **dadurch gekennzeichnet, dass** der Stent ausdehnbar ist und zwar mittels Einwirkung einer radial nach außen gerichteten Kraft, um eine plastische Verformung durchzumachen bis zu einer maximalen Fließgrenze auszuführen, an der die hülsenförmige Wand einen Durchmesser von weniger oder gleich ungefähr 3,5 mm hat.

2. Der unausgedehnte Stent gemäß Anspruch 1, wobei der Stent ausdehnbar ist bei der Einwirkung einer radial nach außen gerichteten Kraft um eine plastische Verformung bis zu einer maximalen Fließgrenze auszuführen, an der die hülsenförmige Wand einen Durchmesser von weniger oder gleich ungefähr 3,3 mm hat.

3. Der unausgedehnte Stent gemäß Anspruch 1, wobei der Stent ausdehnbar ist bei der Einwirkung einer radial nach außen gerichteten Kraft um eine plastische Verformung bis zu einer maximalen Fließgrenze auszuführen, an der die hülsenförmige Wand einen Durchmesser in der Größenordnung von ungefähr 2,2 mm bis zu ungefähr 3,3 mm hat.

4. Der unausgedehnte Stent gemäß Anspruch 1, wobei der Stent ausdehnbar ist bei der Einwirkung einer radial nach außen gerichteten Kraft um eine plastische Verformung bis zu einer maximalen Fließgrenze auszuführen, an der die hülsenförmige Wand einen Durchmesser in der Größenordnung von ungefähr 2,2 bis ungefähr 3,3 mm hat.

5. Der unausgedehnte Stent gemäß einem der Ansprüche 1 - 4, wobei der Stent einen Durchmesser von weniger oder gleich ungefähr 1,1 mm hat.

6. Der unausgedehnte Stent gemäß einem der Ansprüche 1 - 4, wobei der Stent einen Durchmesser in der Größenordnung von ungefähr 0,5 mm bis ungefähr 1,1 mm hat.

7. Der unausgedehnte Stent gemäß einem der Ansprüche 1 - 4, wobei der Stent einen Durchmesser in der Größenordnung von ungefähr 0,5 bis ungefähr 1,0 mm hat.

8. Der unausgedehnte Stent gemäß einem der Ansprüche 1 - 7, wobei die hülsenförmige Wand einen im Wesentlichen kreisförmigen Querschnitt hat.

9. Der unausgedehnte Stent gemäß einem der Ansprüche 1 - 8, wobei die hülsenförmige Wand aus einem plastisch verformbaren Material gebildet ist.

10. Der unausgedehnte Stent gemäß einem der Ansprüche 1 - 9, wobei auf den Stent ein Beschichtungsmaterial aufgebracht ist, welches aus einem oder mehreren biologisch trägen, einer medizinischen Zusammensetzung oder einer radioaktiven Zusammensetzung besteht.

11. Ein teilweise ausgedehnter Stent, umfassend ein nahes und ein distales Ende, die mit einander in Verbindung stehen, sowie eine hülsenförmige Wand, die sich zwischen dem nahen und dem distalen Ende befindet und die eine Längsachse und ein poröse Oberfläche hat, die durch eine Mehrzahl von miteinander verbundenen Verstrebungen gebildet ist, **gekennzeichnet dadurch, dass** der Stent
durch die Anwendung einer radial nach außen wirkenden Kraft hierauf aus einer nicht ausgedehnten Position in eine zweite, vorausgedehnte Position ausgedehnt wurde, bei welcher der Stent einen Punkt einer plastischen Verformung erreicht hat, und
bei Aufbringen einer radial nach außen wirkenden Kraft hierauf von der zweiten, vor-ausgedehnten Position zu einer dritten ausgedehnten Position ausgedehnt wird, wobei der Stent eine plastische Verformung bis zu einem maximalen Endpunkt ausführt, wobei die hülsenförmige Wand von weniger oder gleich etwa 3,5 mm aufweist.

12. Der teilweise ausgedehnte Stent gemäß Anspruch 11, wobei in der dritten ausgedehnten Position des Stents die maximale Fließgrenze erreicht ist, wenn die hülsenförmige Wand einen Durchmesser in der Größenordnung von ungefähr 2,2 bis ungefähr 3,3 mm hat.

13. Der teilweise ausgedehnte Stent gemäß Anspruch 11, wobei in der dritten ausgedehnten Position des Stents die maximale Fließgrenze erreicht ist, wenn die hülsenförmige Wand einen Durchmesser in der Größenordnung von ungefähr 2,2 bis ungefähr 3,3 mm hat.

14. Der teilweise ausgedehnte Stent gemäß Anspruch 11, wobei in der dritten ausgedehnten Position des Stents die maximale Fließgrenze erreicht ist, wenn die hülsenförmige Wand einen Durchmesser in der Größenordnung von ungefähr 2,5 bis ungefähr 3,0 mm hat.

15. Ein Stentbausatz, der beinhaltet:
ein Katheter,
ein ausdehnbares Glied, das an dem Katheter angebracht ist; und der
teilweise ausgedehnte Stent wie in einem der Ansprüche 11 - 14 beschrieben, die auf dem Katheter angebracht ist.

16. Der Stentbausatz gemäß Anspruch 15, wobei der Stent mechanisch auf das ausdehnbare Glied montiert ist.

17. Der Stentbausatz gemäß Anspruch 16, wobei der Stent auf das ausdehnbare Glied gequetscht ist.

## Revendications

1. Un expanseur vasculaire non dilaté comprenant une extrémité proximale et une extrémité distale en communication réciproque, une paroi tubulaire disposée entre l'extrémité proximale et l'extrémité distale, la paroi tubulaire ayant un axe longitudinal et une surface poreuse définie par une pluralité de tiges inter-connectées, **caractérisé en ce que** l'expanseur vasculaire est dilatable par application d'une force radiale dirigée vers l'extérieur en subissant une déformation plastique jusqu'à une limite plastique maximale lorsque la paroi tubulaire présente un diamètre inférieur ou égal à environ 3,5 mm.

2. L'expanseur vasculaire non dilaté selon la revendication 1, dans lequel l'expanseur vasculaire est dilatable par application d'une force radiale dirigée vers l'extérieur sur celui-ci en subissant une déformation plastique jusqu'à une limite plastique maximum lorsque la paroi tubulaire présente un diamètre inférieur ou égal à environ 3,3 mm.

3. L'expanseur vasculaire non dilaté selon la revendication 1, dans lequel l'expanseur vasculaire est dilatable par application d'une force radiale dirigée vers l'extérieur sur celui-ci en subissant une déformation plastique jusqu'à une limite plastique maximum lorsque la paroi tubulaire présente un diamètre dans la fourchette d'environ 2,2 à environ 3,3 mm.

4. L'expanseur vasculaire non dilaté selon la revendication 1, dans lequel l'expanseur vasculaire est dilatable par application d'une force radiale dirigée vers l'extérieur sur celui-ci en subissant une déformation plastique jusqu'à une limite plastique maximum lorsque la paroi tubulaire présente un diamètre dans la fourchette d'environ 2,5 à environ 3,0 mm.

5. L'expanseur vasculaire non dilaté selon une quelconque des revendications 1 à 4, dans lequel l'expanseur vasculaire présente un diamètre inférieur ou égal à environ 1,1 mm.

6. L'expanseur vasculaire non dilaté selon une quelconque des revendications 1 à 4, dans lequel l'expanseur vasculaire présente un diamètre dans la fourchette d'environ 0,5 à environ 1,1 mm.

7. L'expanseur vasculaire non dilaté selon une quelconque des revendications 1 à 4, dans lequel l'expanseur vasculaire présente un diamètre dans la fourchette d'environ 0,5 à environ 1,0 mm.

8. L'expanseur vasculaire non dilaté selon une quelconque des revendications 1 à 7, dans lequel la paroi tubulaire présente une section transversale sensiblement circulaire.

9. L'expanseur vasculaire non dilaté selon une quelconque des revendications 1 à 8, dans lequel la paroi tubulaire est construite en un matériau déformable de manière plastique.

10. L'expanseur vasculaire non dilaté selon une quelconque des revendications 1 à 9, dans lequel l'expanseur vasculaire porte une matière de revêtement choisie parmi une ou plusieurs du groupe constitué d'une matière biologiquement inerte, d'une composition médicinale et d'une composition radioactive.

11. Un expanseur vasculaire partiellement dilaté comprenant une extrémité proximale et une extrémité distale communiquant l'une avec l'autre, une paroi tubulaire disposée entre l'extrémité proximale et l'extrémité distale, la paroi tubulaire ayant un axe longitudinal et une surface poreuse définie par une pluralité de tiges interconnectées, **caractérisé en ce que** l'expanseur vasculaire :
a été dilaté par l'application d'une force radiale dirigée vers l'extérieur sur celui-ci à partir d'une première position non dilatée à une seconde position pré-dilatée à laquelle l'expanseur vasculaire a atteint un point de déformation plastique, et
est susceptible d'être dilaté encore par application d'une force radiale dirigée vers l'extérieur sur celui-ci à partir de la seconde position pré-dilatée à une troisième position dilatée dans laquelle l'expanseur vasculaire va subir une déformation plastique jusqu'à une limite plastique maximum lorsque la paroi tubulaire a un diamètre inférieur ou égal à environ 3,5 mm.

12. L'expanseur vasculaire partiellement dilaté selon la revendication 11, dans lequel, dans la troisième position dilatée du expanseur vasculaire, la limite plastique maximum est atteinte lorsque la paroi tubulaire présente un diamètre inférieur ou égal à environ 3,3 mm.

13. L'expanseur vasculaire partiellement dilaté selon la revendication 11, dans lequel, dans la troisième position dilatée du expanseur vasculaire, la limite plastique maximum est atteinte lorsque la paroi tubulaire présente un diamètre dans la fourchette d'environ 2,2 à environ 3,3 mm.

14. L'expanseur vasculaire partiellement dilaté selon la revendication 11, dans lequel, dans la troisième position dilatée du expanseur vasculaire, la limite plastique maximum est atteinte lorsque la paroi tubulaire présente un diamètre dans la fourchette d'environ 2,5 à environ 3,0 mm.

15. Une panoplie d'application d'un expanseur vasculaire comprenant :
- un cathéter ;
- un élément dilatable disposé sur le cathéter ; et
- l'expanseur vasculaire partiellement dilaté selon l'une quelconque des revendications 11 à 14 disposé sur le cathéter.

16. La panoplie d'application de expanseur vasculaire selon la revendication 15, dans laquelle l'expanseur vasculaire est monté mécaniquement sur l'élément dilatable.

17. La panoplie d'application de expanseur vasculaire selon la revendication 16, dans laquelle l'expanseur vasculaire est serti sur l'élément dilatable.
